# EUROPEAN PATENT APPLICATION

(11) **EP 2 052 741 A1**
(43) Date of publication of application: **29.04.2009**
(21) Application number: 07021035.6
(22) Date of filing: 26.10.2007
(51) Int. Cl.: A61K 51/10

(54) **Radioactive material for inhibiting cancer and preparation method thereof**

(71) Applicant: Atomic Energy Council - Institute of Nuclear Energy Research, Taoyuan County (TW)
(72) Inventor: Tang, I-Chung, Longtan Township Taoyuan County (TW); Luo, Tasi-Yueh, Longtan Township Taoyuan County (TW); Wu, Yu-Lung, Longtan Township Taoyuan County (TW); Sheng, Chang-Mau, Longtan Township Taoyuan County (TW); Cheng, Shan-Yun, Longtan Township Taoyuan County (TW); Liu, Hsiu-Wen, Longtan Township Taoyuan County (TW); Yeh, Yuen-Han, Longtan Township Taoyuan County (TW); Lin, Wuu-Jyh, Longtan Township Taoyuan County (TW)
(74) Representative: Kador & Partner

(57) **Abstract**

A radioactive material for inhibiting cancer and a preparation method thereof are disclosed. The radioactive material for inhibiting cancer is M-SOCTA-Z and M is a radioactive nuclide such as ¹⁸⁸Re or ^{99m}T while Z is protein or peptides having amino acid with NH or NH2 group. The preparation method of the radioactive material for inhibiting cancer includes steps of: reacting SOCTA with protein or peptide. Ester (-COOR) in SOCTA reacts with amines (-NH, -NH2) in protein or peptide to form peptide bond. Thus SOCTA-protein or SOCTA-peptide complex is produced. Then these SOCTA-protein complex reacts with radioactive nuclide M so as to generate M-SOCTA-protein or M-SOCTA-peptide. In an embodiment of the present invention, monoclonal antibody Herceptin is applied to bind with SOCTA and in combination with radiation-based therapy, effects of cancer treatment (such as breast cancer) are enhanced.

## Description

### BACKGROUND OF THE INVENTION

The present invention relates to a radioactive material for inhibiting cancer and a preparation method thereof, especially to a radioactive material ¹⁸⁸Re-SOCTA-Herceptin and a preparation method thereof that are applied to inhibit growth of cancer cells such as breast cancer cells and destroy cancer cells so as to improve outcomes of breast cancer.

Women without periods or older than 40 years have higher probability to get cancer such as breast cancer, ovary cancer and cervix cancer due to change of hormone in blood. Besides single organ (breast), the cancer may also invade other organs such as bones, liver, lung and brain, destroy fetal functions of bodies and even threaten lives. According to statistics of Department of Health, incidence and mortality of breast cancer is getting increasing year after year. In 2005, breast cancer was the fourth leading cause of death for women in Taiwan, and the biggest killer in western countries. Breast cancer brings patents and their families heavy physical and metal loads. Thus it has become an important issue in public health.

Conventional therapies for breast cancer include surgery, adjuvant drug therapy, hormone therapy and actinothreapy. In recent years, cancer research has resulted in a breakthrough treatment for breast cancer - antibody therapy. This is by means of monoclonal antibody Herceptin to against Her-2/neu (or Erb B-2) antigen in cancer cells.

In 1983, Hayman etc. reported that in sarcoma or erythroblastosis cells of chicken, an oncogen producing glycoprotein is found and named erb-B2. In 1986, Bargman et al. also found an oncogen named neu (erb-B2) in neuroblastoma of rats, similar to the oncogen in erythroblastosis. Another oncogen that is quite similar to EGFR gene in normal human cells named HER-2 (human epidermal growth factor receptor-2) is found. Thus the 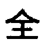 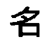 of the oncogene is C-erb-B2/Her-2/neu, Her-2/neu for short. The protein generated by this gene is p185HER-2. In cancer cells of breast cancer patients, about 30% has mutation in Her-2/neu. Therefore, this oncogene is an important marker for confirming breast cancer cells. In other kind of cancers such as non-small cell lung cancer, gastric cancer, prostate cancer, ovarian cancer, and cervical cancer etc., HER-2/neu is expressed in high level. In some kind of cancers, high level expression of HER-2/neu is an important prognostic factor.

Baselga et al apply Herceptin to medical research on human subjects. Herceptin is used to treat 46 patients with advanced breast cancer as well as over-expression of HER2 and the response rate is 11.6%. Then a secondary clinical trial with larger scale is conducted. 222 patients with no response to chemotherapy are treated by Herceptin, the response rate 16% is and the control period is 9.1 months. The 16% response rate may be considered too low. However, all patients in the trial are not be as responsive to various drugs in chemotherapy and the cancer cells exhibit a high level of robustness against a range of therapeutic efforts. Thus such result is a kind of breakthrough. Due to unique effect and action mechanism of Herceptin, it's a drug with priority review and has been approved for use by the U.S. Food and Drug Administration (FDA) in September 1998, It's used to treat women with HER2-overexpressing metastatic breast cancer and therapy failure to at least one drug in chemotherapy. The present invention uses Herceptin, together with radioimmunotherapy (RIT). High-energy β particles of 188Re are therapeutically effective to destroy breast cancer cells and thus the breast cancer treatment is improved.

Herceptin is effective for the treatment of patients with Her-2/neu over-expressing. As a single agent, Herceptin has about 30% tumor response rate and it can inhibit dimerization and phosphorylation of Her-2/neu receptor so as to delay growth of tumors and even destroy them. Herceptin in combination with conventional chemotherapy such as anthracyclines and cyclophosphamide, is indicated for the treatment of patients with metastatic breast cancer, also with enhanced therapeutic effects.

As a generator-produced radioisotope emitting both beta (2.2MeV) and gamma rays (155keV) and having a short physical half-life of 16.9 h, rhenium-188 from β-decay of ¹⁸⁸W is a very good potential candidate for diagnosis and therapy. SOCTA (succinimidy-3,6-diaza-5-oxo-3-[2-((tri-phenylmethyl)thio)ethyl]-8-[(trip he- nylmethyl)thio]octanoate)], is a new bi-functional organic ligand invented by Dr. Cheng-Hsien Lin, in Institute of Nuclear Energy Research, Taiwan. In structure of SOCTA, an activated carboxylic acid is used to bind with protein or peptide (in present invention the protein is monoclonal antibody Herceptin). Morever, the SOCTA has N₂S₂ ligand to bind with rhenium and technetium. Once the protein or peptide is intended to be labeled with rhenium and technetium, compound SOCTA is a good choice.

In order to enhance anti-cancer effects of monoclonal antibody Herceptin, the present invention provides a radioactive material for inhibiting cancer and a preparation method thereof. Herceptin is covalently bound with SOCTA and then SOCTA-Herceptin is labeled with radioisotope M such as ¹⁸⁸Re to form such structure ¹⁸⁸Re-SOCTA-Herceptin. Because Herceptin inhibits expression of Her-2/neu oncogene on breast cancer cells, together with 2.2MeV β particle emitted from ¹⁸⁸Re of the compound -¹⁸⁸Re-SOCTA-Herceptin, and penetrating up to 6.4 - 11 mm in tissue, growth of cancer cells such as breast cancer is inhibited and the cancer cell is even destroyed so as to achieve effective cancer therapy.

### SUMMARY OF THE INVENTION

Therefore it is a primary object of the present invention to provide a radioactive material for inhibiting cancer and a preparation method thereof. The radioactive material consists of radioactive elements and monoclonal anotibody Herceptin so as to inhibit growth of tumor (such as breast cancer) and destroy cancer cells for enhancing effects of caner therapy.

It is another object of the present invention to provide a radioactive material for inhibiting cancer and a preparation method thereof. The preparation method of the radioactive material for inhibiting cancer is one stage synthesis that shortens synthesis time of cancer inhibitor such as ¹⁸⁸Re-SOCTA-Herceptin and increases yield rate.

In order to achieve above objects, a radioactive material for inhibiting cancer according to the present invention having: 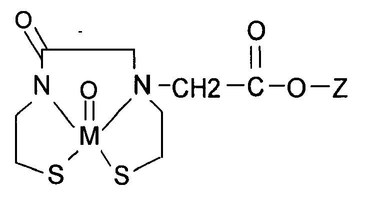 wherein M is selected from a group of ¹⁸⁸Re and ^{99m}Tc while Z is selected from a group consisting of protein having amino acid with amino group and peptide having amino acid with amino group.

A preparation method of the radioactive material for inhibiting cancer according to the present invention includes following steps: react SOCTA with protein or peptide to form SOCTA complex. The SOCTA complex is SOCTA-protein complex or SOCTA-peptide complex. Then the SOCTA complex reacts with material M to form M-SOCTA complex while M is selected from a group of ¹⁸⁸Re and ^{99m}Tc. And the M-SOCTA complex is selected from a group consisting of ¹⁸⁸Re-SOCTA-protein complex, ¹⁸⁸Re-SOCTA-peptide complex, ^{99m}Tc -SOCTA-protein complex, and ^{99m}Tc -SOCTA-peptide complex. The M-SOCTA complex is a radioactive material that inhibits cancers.

### BRIEF DESCRIPTION OF THE DRAWINGS

The structure and the technical means adopted by the present invention to achieve the above and other objects can be best understood by referring to the following detailed description of the preferred embodiments and the accompanying drawings, wherein
Fig. 1 shows chemical structure of SOCTA in an embodiment of the present invention;
Fig. 2 is a flow chart of a preparation method of radioactive material for inhibiting cancer according to the present invention;
Fig. 3 is a graph showing radiochemical purity of ¹⁸⁸Re-Glucoheptonate versus reaction time curve of an embodiment according to the present invention;
Fig. 4 shows the labeling efficiency of ¹⁸⁸Re-Glucoheptonate versus reaction time curves obtained at three different temperatures;
Fig. 5 shows the labeling efficiency versus reaction time curves obtained by addition of different amount of SnCl₂;
Fig. 6 shows the labeling efficiency versus reaction time curves obtained by addition of different volume of SOCTA-Herceptin;
Fig. 7 shows the labeling efficiency versus reaction time curve of performing stability test in serum.

### DETAILED DESCRIPTION OF THE PREFERRED EMBODIMENT

A radioactive material for inhibiting cancer according to the present invention having: 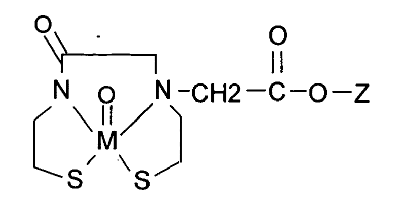 wherein M is selected from a group of ¹⁸⁸Re and ^{99m}Tc while Z is selected from a group consisting of protein having amino acid with amino group and peptide having amino acid with amino group. The amino group is selected from a group of -NH and -NH₂. The amino acid with amino group is selected from a group consisting of Lysine, Arginine, Glutamine, and Asparagine. The protein having amino acid with amino group is a monoclonal antibody and is a monoclonal antibodyHerceptin.

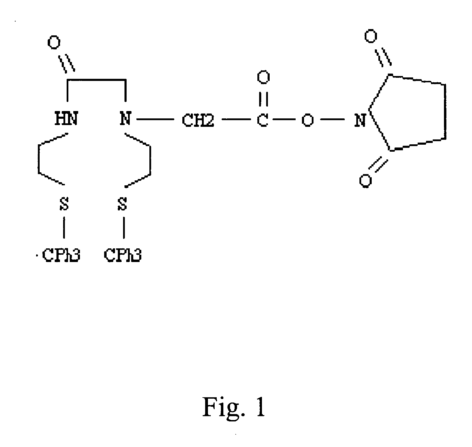

Refer to Fig. 1, is a new organic ligand with bifunctional groups-SOCTA synthesis by the present invention includes an activated carboxylic acid for bonding with protein or peptide. In the present invention, it's bonded with monoclonal antibody Herceptin. Moreover, another ligand N₂S₂ in SOCTA is used to bond with rhenium and technetium. Thus compound SOCTA can be used to label protein or peptide with rhenium or technetium.

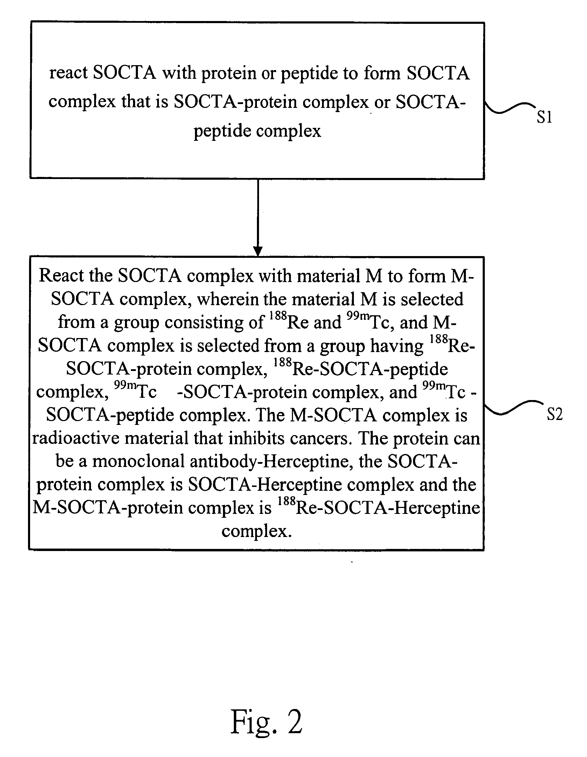

The preparation method of the radioactive material for inhibiting cancer according to the present invention includes following steps, as shown in Fig. 2:
S 1 react SOCTA with protein or peptide to form SOCTA complex that is SOCTA-protein complex or SOCTA-peptide complex;
S2 React the SOCTA complex with material M to form M-SOCTA complex, wherein the material M is selected from a group consisting of ¹⁸⁸Re and ^{99m}Tc, and M-SOCTA complex is selected from a group having ¹⁸⁸Re-SOCTA-protein complex, ¹⁸⁸Re-SOCTA-peptide complex, ^{99m}Tc -SOCTA-protein complex, and ^{99m}Tc -SOCTA-peptide complex. The M-SOCTA complex is radioactive material that inhibits cancers. The protein can be a monoclonal antibody-Herceptine, the SOCTA-protein complex is SOCTA-Herceptine complex and the M-SOCTA-protein complex is ¹⁸⁸Re-SOCTA-Herceptine complex.

In the step S1, ester (-COOR) in SOCTA reacts with amines (-NH, -NH2) in protein or peptide to form peptide bond. Thus SOCTA-protein or SOCTA-peptide complex is produced. In the step of reacting SOCTA with Herceptin to form SOCTA-Herceptin complex, coupling reaction is carried out between SOCTA and Herceptin. The step S 1 further includes a step of dissolving SOCTA in dimethylformamide (DMF) solution as well as dissolving the Herceptin in HEPES (4-(2-hydroxyethyl)-1-piperazineethanesulfonic acid) buffer. The preferred molecular ratio of SOCTA to Herceptin is 1:1. The step of reacting SOCTA with Herceptin to form SOCTA-Herceptin is carried out at room temperature. Moreover, the step S1 further includes a step of dialysis.

In the step S2, SOCTA-Herceptin complex reacts with ¹¹⁸Re to form ¹⁸⁸Re-SOCTA-Herceptin while ¹⁸⁸Re is provided by ¹⁸⁸Re perrhenate solution. The step is two-stage. Firstly, ¹⁸⁸Re , glucoheptonate and stannous chloride (SnCl₂), react with one another to produce ¹⁸⁸Re-Glucoheptonate. Then the ¹⁸⁸Re-Glucoheptonate reacts with SOCTA-Herceptin to get ¹⁸⁸Re-SOCTA-Herceptin. In the first stage, weight ratio of glucoheptonate to stannous chloride is from 1.5 : 1 to 5.4 : 1 while 1.6 : 1 is preferably. The glucoheptonate is a chelating agent. The reaction temperature of the step of reacting ¹⁸⁸Re-Glucoheptonate with SOCTA-Herceptin to get ¹⁸⁸Re-SOCTA-Herceptin is room temperature or 37 degrees Celsius (□).

In the step S2, it can also be a one stage step that ¹⁸⁸Re , glucoheptonate, stannous chloride (SnCl₂), and SOCTA-Herceptin react with one another to produce ¹⁸⁸Re-SOCTA-Herceptin. The weight ratio of glucoheptonate to stannous chloride is from 1.5 : 1 to 5.4 : 1 while 1.6 : 1 is preferably. The preferred reaction temperature is room temperature or 37□.

### SOCTA-Herceptin coupling reaction

Dissolve certain amount of SOCTA in DMF solution. Dissolve certain amount of Herceptin in HEPES Buffer while the HEPES Buffer is made by dissolving HEPES and 0.29g sodium chloride (NaCl) in water (pH=7.4). Take certain amount of SOCTA solution and Herceptin solution with molecular ratio of 1:1 and put them into 1.5ml centrifuge tube, reacting for 3 hours. Remove the reacted solution into a dialysis bag, sealed the bag, put into a refrigerator at 4 4□ and dialysis for two days. The Dialysis Buffer consisting of sodium citrate, sodium chloride, dissolved in 2-liter water and the solution is adjusted to PH 5.2. After dialysis, the solution inside the bag is centrifuged at 4□ for 10 minutes. Collect the supernatant.

The molecular ratio of SOCTA to Herceptin in this reaction is 1:1. After coupling reaction, the dialyzed SOCTA-Herceptin complex is measured by enzyme-link immunosorbent assay (ELISA). After detecting absorption values of standard sample and unknown sample by ELISA, the concentration of the protein get by excel interpolation is 7.9mg/ml.

### Two-stage preparation of ¹⁸⁸Re-SOCTA-Herceptin

### 1. Preparation of ¹⁸⁸Re-Glucoheptonate

Take 400µl ¹⁸⁸Re perrhenate solution, 6-14 mg stannous chloride and glucoheptonate (GH), adding into a 1.5ml centrifuge tube and react at room temperature. After mixing, take samples for analysis of radiochemical purity at the 15^{th} minute. The analysis method uses miniaturized instant TLC plates impregnated with silica gel (ITLC-SG) for the stationary phase and normal saline with Methylethylketone (MEK) for the mobile phase.

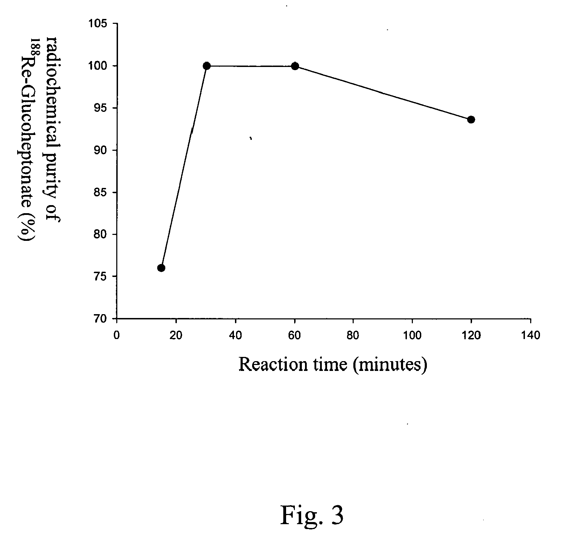

The glucohepatonate (GH) is used as weak chelating agent. Add 32mg GH, 6mg SnCl₂, and ¹⁸⁸Re perrhenate solution into a reaction vessel, react at room temperature and take samples at proposed time for radiochemical analysis, as shown in Fig. 3. The result shows that at the 15^{th} minute, radiochemical purity of ¹⁸⁸Re-GH achieves 76% while a radiochemical purity of 100% is achieved at the 30^{th} minute and remains at the 60^{th} minute. It is sure that after reacting for 30 to 60 minutes, ¹⁸⁸Re-GH achieves preset radiochemical standard. This reaction condition is selected and is provided for ¹⁸⁸Re-GH to react with SOCTA- Herceptin at the next stage.

### 2. Prepation of ¹⁸⁸Re-SOCTA-Herceptin

The ¹⁸⁸Re-GH solution with radiochemical purity of 100% is added with certain amount of SOCTA-Herceptin and reacting continuously. In order to evaluate the effects of reaction temperature on the labeling efficiency, two different temperatures are used in this experiment-room temperature and 37□. After beginning of reaction, take samples for analysis at the 15^{th}, 30^{th}, 60^{th}, 90^{th} and 120^{th} minutes. Perform radiochemical purity analysis by ITLC-SG/MEK or NS system together with radio-thin-layer chromatography (Radio-TLC).

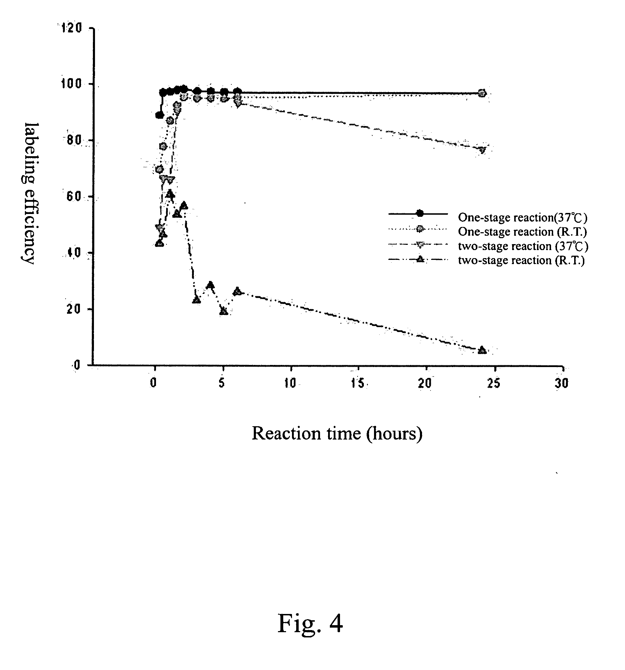

After reaction finishing, the ¹⁸⁸Re-GH is added with 20µl SOCTA-Herceptin. The experiment includes two groups -room temperature and 37□ so as to learn effects of the temperature on the labeling efficiency. Refer to Fig. 4, the result show that under reaction condition of 37□, labeling efficiency of the products increases from 48.99% at a half hour to 66.12% at the first hour and maximum achieves 96.01±0.13% at the second hour.

While reacting at room temperature, the ¹⁸⁸Re-GH added with SOCTA-Herceptin is also taken samples at different times for radiochemical purity analysis. The result show that the maximum labeling efficiency at the first hour is only 60.83±8.59%.

### One-stage preparation of ¹⁸⁸Re-SOCTA-Herceptin

Add ¹⁸⁸Re perrhenate solution, SnCl₂, Glucoheptonate and SOCTA-Herceptin into a tube simultaneously. Then the react at room temperature and 37□ respectively to observe change of radiochemical purity at different times. The method of radiochemical purity analysis is as two-stage by ITLC-SG/MEK (or NS) system.

Although two-stage preparation method is able to achieve 96% radiochemical purity at of 37°C, the reaction is time consuming. Thus it's replaced by one-stage method. Take certain amount of ¹⁸⁸Re , glucoheptonate, stannous chloride (SnCl₂), and SOCTA-Herceptin into a reaction vial. In order to evaluate effects of certain conditions such as reaction temperature, amount of stannous chloride and amount of SOCTA-Herceptin on the labeling efficiency, some tests are designed and implemented and the results are as following:

### 1.effect of the temperature on the labeling efficiency

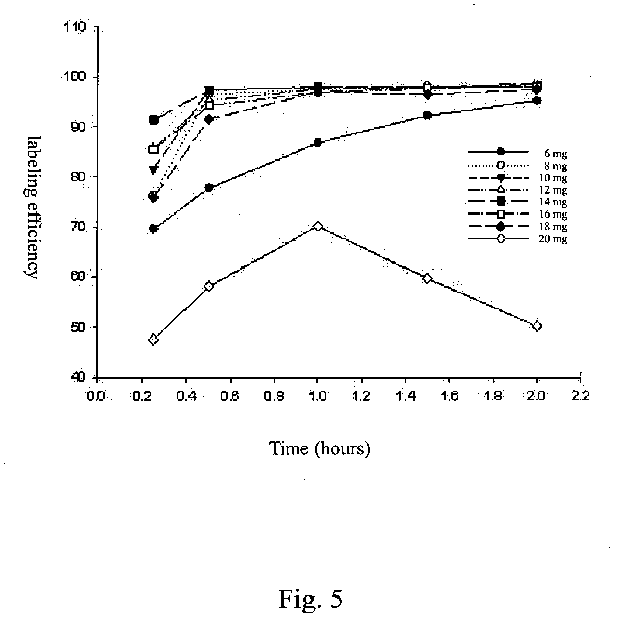

Firstly, a condition of 37°C is applied and it is found that the labeling efficiency achieves 96.91% after a half hour. Continuingly observe 24 hours, the radiochemical purity of ¹⁸⁸Re-SOCTA-Herceptin remains 97%, as shown in Fig. 4. At the same time, reaction at room temperature is given. Refer to Fig. 5, when the amount of SnCl₂ is 6mg, the radiochemical purity of ¹⁸⁸Re-SOCTA-Herceptin increases along with reaction time. At the second hour, the labeling efficiency achieves 95.18±0.50%. When the amount of SnCl₂ is over 8mg, the labeling efficiency achieves 95% only after a half hour.

### 2.effect of the amount of stannous chloride on the labeling efficiency

Stannous chloride is the most common reducing agent in medical field. In the present embodiment, is SnCl₂ used to reduce ¹⁸⁸Re (VII) into ¹⁸⁸Re (V). Refer to Fig. 5, compare effects of various amount of SnCl₂ such as 6, 8, 10, 12, 14, 16, 18 and 20mg on the labeling efficiency at room temperature. It is found that the labeling efficiency of the SOCTA-Herceptin achieves 91 % at the 15^{th} minute by addition of 14mg SnCl₂ while the labeling efficiency achieves 98.71 % at the second hour. The labeling efficiency of the curve with addition of 14mg SnCl₂ at each time point is larger than that of the curve with addition of 6mg SnCl₂. The difference is significant. Once the amount of SnCl₂ added is over 8mg, the efficiency is 95% after reacting a half hour at room temperature and the efficiency remains at least two hours. Although the result is good, it is easy to produce gel. Thus the reaction condition with 6mg SnCl₂ and at room temperature is selected for the reaction of labeling ¹⁸⁸Re-SOCTA-Herceptin.

### 3.effect of the volume of SOCTA-Herceptin on the labeling efficiency

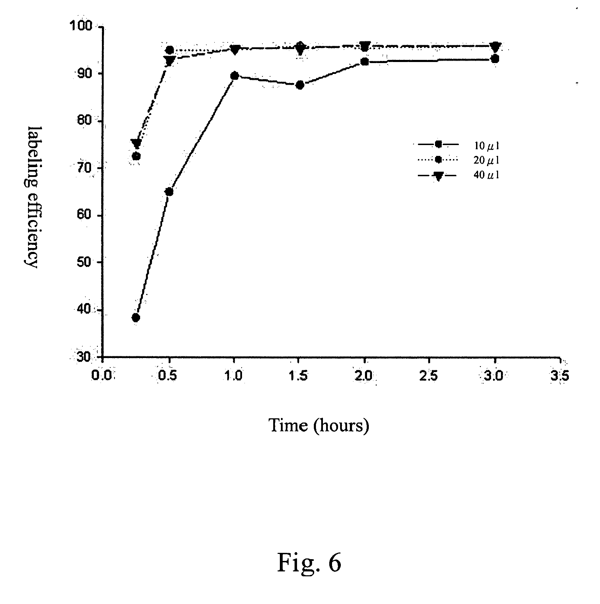

Three kinds of volume of SOCTA-Herceptin-10, 20 and 40 µ*l* are selected, as shown in Fig. 6. There is 0.158mg SOCTA-Herceptin in 20µ*l* SOCTA-Herceptin solution so that 10µ*l* solution has 0.079mg. The labeling efficiency of 10µ*l* SOCTA-Herceptin reacting at room temperature for 0.25 and 0.5 hour is 38.29% and 65.01% respectively. The curve of 10µ*l* is significantly difference from curves of 20 and 40 µ*l*. The maximum labeling efficiency occurs at the third hour with the value of 93.30%. Once adding 20 or 40 µ*l* of SOCTA-Herceptin, the average labeling efficiency achieves at least 93% at the 30^{th} minute. The curve of 20µ*l* is quite similar to that of 40µ*l*.

### In vitro stability test of ¹⁸⁸Re-SOCTA-Herceptin

There are two groups. On is to put ¹⁸⁸Re-SOCTA- Herceptin at room temperature and observe change of radiochemical purity at different time points. The other group is adding ¹⁸⁸Re-SOCTA-Herceptin into human serum, react at 37°C, and observe change of radiochemical purity along with time.

After finishing labeling of ¹⁸⁸Re-SOCTA-Herceptin, set the product at room temperature for performing 24-hour stability test of the product radiochemical purity. The test result shows that the labeling efficiency of ¹⁸⁸Re-SOCTA-Herceptin stably maintains from 94% to 96% since the second hour to the 24^{th} hour.

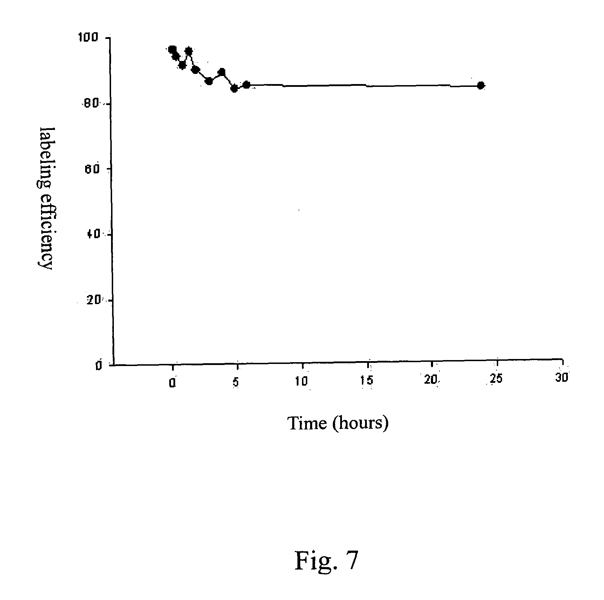

The stability in the serum us also evaluated. After finishing labeling, the ⁸⁸Re-SOCTA-Herceptin is added in human serum at 37□. Then perform 24-hour stability test of the product radiochemical purity. The test result shows that the labeling efficiency of ¹⁸⁸Re-SOCTA-Herceptin reduces gradually to 90% at the second hour and maintains at 83% until the 24^{th} hour, as shown in Fig. 7.

In the beginning, the two-stage labeling way is to react ¹⁸⁸Re with Glucoheptonate to form complex compound. Then through ligand-exchange, GH is replaced by the SOCTA-Herceptin. The one-stage labeling method save about 1 to 1.5 hour on labeling time and there is not need to heat. At average room temperature of 22□ 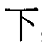 , it takes about one hour to finish labeling. As to radioisotope for therapeutic applications, only labeling time is saved but also treatment time is extended. As to stability, two-stage labeling is gradually reduced form the second hour after labeling and only 77.29% at the 24^{th} hour while one-stage keeps 96.88%.

The one-stage labeling takes the shortest time with highest labeling efficiency and it maintains stability for 24 hours. Without filtering procedures, the labeling efficiency and stability of the present invention are over 95% and maintains for 24 hour, the purity is near 100%. The results show that nearly 100% purity of ¹⁸⁸Re-SOCTA-Herceptin with high labeling efficiency and 24-hour stability is produced. In futures, it is applied to cells and animal models for evaluation of therapeutic effects for providing better clinical treatment of cancers such as breast cancer.

In summary, a preparation method of the radioactive material for inhibiting cancer according to the present invention is a one-stage step that reduces synthesis time of radioactive material such as ¹⁸⁸Re-SOCTA-Herceptin and increase yield rate thereof. The radioactive material for inhibiting cancer according to the present invention includes radioactive element and monoclonal antibody Herceptin so that it enhances inhibition of growth of breast cells such as breast cancer cells and destruction of cancer cells.

Additional advantages and modifications will readily occur to those skilled in the art. Therefore, the invention in its broader aspects is not limited to the specific details, and representative devices shown and described herein. Accordingly, various modifications may be made without departing from the spirit or scope of the general inventive concept as defined by the appended claims and their equivalents.

## Claims

1. A radioactive material for inhibiting cancer comprising: 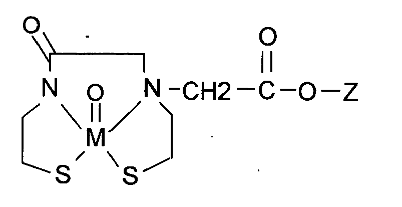 wherein M is selected from a group of ¹⁸⁸Re and ^{99m}Tc while Z is selected from a group consisting of protein having amino acid with amino group and peptide having amino acid with amino group.

2. The radioactive material for inhibiting cancer as claimed in claim 1, wherein the amino group is selected from a group of -NH and -NH₂.

3. The radioactive material for inhibiting cancer as claimed in claim 1, wherein the amino acid with amino group is selected from a group consisting of Lysine, Arginine, Glutamine, and Asparagine.

4. The radioactive material for inhibiting cancer as claimed in claim 1, wherein the protein having amino acid with amino group is a monoclonal antibody.

5. The radioactive material for inhibiting cancer as claimed in claim 4, wherein the monoclonal antibody is Herceptin.

6. A preparation method of the radioactive material for inhibiting cancer comprising the steps of:
reacting SOCTA with protein or peptide to form SOCTA complex while the SOCTA complex is SOCTA-protein complex or SOCTA-peptide complex; and
reacting the SOCTA complex with material M to form M-SOCTA complex while M is selected from a group of ¹⁸⁸Re and ^{99m}Tc, and the M-SOCTA complex is selected from a group consisting of ¹⁸⁸Re-SOCTA-protein complex, ¹⁸⁸Re-SOCTA-peptide complex, ^{99m}Tc -SOCTA-protein complex, and ^{99m}Tc -SOCTA-peptide complex;
wherein the M-SOCTA complex is a radioactive material for inhibiting cancer.

7. The preparation method of the radioactive material for inhibiting cancer as claimed in claim 6, wherein the protein is a monoclonal antibody Herceptin, the SOCTA-protein complex is SOCTA-Herceptine complex and the M-SOCTA-protein complex is ¹⁸⁸Re-SOCTA-Herceptine complex.

8. The preparation method of the radioactive material for inhibiting cancer as claimed in claim 7, wherein in the step of reacting the SOCTA with the monoclonal antibody Herceptin to form SOCTA-Herceptin complex, a coupling reaction occurs between the SOCTA and the Herceptin.

9. The preparation method of the radioactive material for inhibiting cancer as claimed in claim 7, wherein in the step of reacting the SOCTA with the monoclonal antibody Herceptin to form SOCTA-Herceptin complex, the method further comprising a step of dissolving SOCTA in dimethylformamide (DMF) solution as well as dissolving the Herceptin in HEPES (4-(2-hydroxyethyl)-1-piperazineethanesulfonic acid) buffer.

10. The preparation method of the radioactive material for inhibiting cancer as claimed in claim 7, wherein in the step of reacting the SOCTA with the monoclonal antibody Herceptin to form SOCTA-Herceptin complex, the molecular ratio of SOCTA to Herceptin is 1:1.

11. The preparation method of the radioactive material for inhibiting cancer as claimed in claim 7, wherein the step of reacting the SOCTA with the monoclonal antibody Herceptin to form SOCTA-Herceptin complex is carried out at room temperature.

12. The preparation method of the radioactive material for inhibiting cancer as claimed in claim 7, wherein the step of reacting the SOCTA with the monoclonal antibody Herceptin to form SOCTA-Herceptin complex further comprising a step of dialysis.

13. The preparation method of the radioactive material for inhibiting cancer as claimed in claim 7, wherein in the step of reacting the SOCTA-Herceptin complex with the ¹⁸⁸Re to form the ¹⁸⁸Re-SOCTA-Herceptin, the ¹⁸⁸Re is provided by ¹⁸⁸Re perrhenate solution.

14. The preparation method of the radioactive material for inhibiting cancer as claimed in claim 7, wherein the step of reacting the SOCTA-Herceptin complex with the ¹⁸⁸Re to form the ¹⁸⁸Re-SOCTA-Herceptin further comprising two-stage steps of:
reacting the ¹⁸⁸Re, glucoheptonate and stannous chloride (SnCl₂) with one another to produce ¹⁸⁸Re-Glucoheptonate; and
reacting the ¹⁸⁸Re-Glucoheptonate with SOCTA-Herceptin to get the ¹⁸⁸Re-SOCTA-Herceptin.

15. The preparation method of the radioactive material for inhibiting cancer as claimed in claim 14, wherein in the step of reacting the ¹⁸⁸Re, glucoheptonate and stannous chloride (SnCl₂) with one another to produce ¹⁸⁸Re-Glucoheptonate, weight ratio of the glucoheptonate to the stannous chloride ranges from 1.5 : 1 to 5.4 : 1

16. The preparation method of the radioactive material for inhibiting cancer as claimed in claim 15, wherein in the step of reacting the ¹⁸⁸Re, glucoheptonate and stannous chloride (SnCl₂) with one another to produce ¹⁸⁸Re-Glucoheptonate, weight ratio of the glucoheptonate to the stannous chloride is 1.6 : 1.

17. The preparation method of the radioactive material for inhibiting cancer as claimed in claim 14, wherein in the step of reacting the ¹⁸⁸Re, glucoheptonate and stannous chloride (SnCl₂) with one another to produce ¹⁸⁸Re-Glucoheptonate, the glucoheptonate is a chelating agent.

18. The preparation method of the radioactive material for inhibiting cancer as claimed in claim 14, wherein the step of reacting the ¹⁸⁸Re-Glucoheptonate with SOCTA-Herceptin to get the ¹⁸⁸Re-SOCTA-Herceptin is taken place at room temperature or 37 degrees Celsius.

19. The preparation method of the radioactive material for inhibiting cancer as claimed in claim 7, wherein the step of reacting the SOCTA-Herceptin complex with the ¹⁸⁸Re to form the ¹⁸⁸Re-SOCTA-Herceptin comprising a one-stage step of: reacting ¹⁸⁸Re , glucoheptonate, stannous chloride (SnCl₂), and SOCTA-Herceptin with one another to produce ¹⁸⁸Re-SOCTA-Herceptin.

20. The preparation method of the radioactive material for inhibiting cancer as claimed in claim 19, wherein in the one-stage step of reacting ¹⁸⁸Re, glucoheptonate, stannous chloride (SnCl₂), and SOCTA-Herceptin with one another to produce ¹⁸⁸Re-SOCTA-Herceptin, weight ratio of the glucoheptonate to the stannous chloride is from 1.5 : 1 to 5.4 : 1

21. The preparation method of the radioactive material for inhibiting cancer as claimed in claim 20, wherein in the one-stage step of reacting ¹⁸⁸Re, glucoheptonate, stannous chloride (SnCl₂), and SOCTA-Herceptin with one another to produce ¹⁸⁸Re-SOCTA-Herceptin, weight ratio of the glucoheptonate to the stannous chloride is 1.6 : 1.

22. The preparation method of the radioactive material for inhibiting cancer as claimed in claim 19, wherein the one-stage step of reacting ¹⁸⁸Re, glucoheptonate, stannous chloride (SnCl₂), and SOCTA-Herceptin with one another to produce ¹⁸⁸Re-SOCTA-Herceptin is carried out at room temperature or 37□.

23. The preparation method of the radioactive material for inhibiting cancer as claimed in claim 6, wherein in the step of reacting SOCTA with protein or peptide to form SOCTA complex, ester (-COOR) in the SOCTA reacts with amines (-NH, -NH2) in the protein or peptide to form peptide bond so as to produce the SOCTA complex.
